# EUROPEAN PATENT APPLICATION

(11) **EP 4 220 647 A1**
(43) Date of publication of application: **02.08.2023**
(21) Application number: 21872610.7
(22) Date of filing: 27.09.2021
(51) Int. Cl.: G16H 10/00

(54) **GROUP REGISTRATION DEVICE, GROUP REGISTRATION METHOD, AND GROUP REGISTRATION PROGRAM**

(30) Priority: 28.09.2020 JP 2020162543
(71) Applicant: Tanita Corporation, Tokyo 174-8630 (JP)
(72) Inventor: KONO Rumi, Tokyo 174-8630 (JP); KODAMA Miyuki, Tokyo 174-8630 (JP)
(74) Representative: Eisenführ Speiser
(86) International application number: PCT/JP2021/035371
(87) International publication number: WO 2022/065484

(57) **Abstract**

A group system, based on physical information indicating a physical state of a user, determines a user type representing the physical state of the user from one or more predetermined points of view, and registers a user whose user type has been determined for a group to which another user having a commonality belongs. A user is registered for a group under a determined user type, and therefore can communicate with another user without the user's physical information being known directly to the other user.

## Description

### CROSS-REFERENCE TO RELATED APPLICATION

This application claims the benefit of Japanese Patent Application No. 2020-162543 filed on September 28, 2020 in Japan, the contents of which are incorporated herein by reference.

### TECHNICAL FIELD

The present disclosure relates to a group registration device, a group registration method, and a group registration program.

### BACKGROUND ART

Recently, application software that allows people having the same hobby and acquaintances to form a group and communicate with one another is spread widely.

For example, WO 2007/063605, which is Patent document 1, discloses a behavior improvement system that makes a user work on behavior improvement using a network system that allows for the exchange of information among an organization or a group made up of a plurality of users. This behavior improvement system acquires health condition information indicating the health condition of a user. An analysis is then made of the correlation between the degree of achievement of a result goal or behavior goal and the daily health condition. If the behavior improvement system consequently obtains an analysis result where, for example, a user with a high degree of achievement or a high scorer is in a stable health condition, the system recommends each user to care about their health condition.

### SUMMARY OF THE INVENTION

### Problems to be solved by the invention

Now, when a user joins a group aiming for dieting or other health promotion, the user's own actual body shape and age may be required to be disclosed. The disclosure of a user's own actual body shape and age would allow for sharing of the body shape, a change in value related to the body shape, or the like within the group, and this increases a sense of unity among the users within the group and promises an effect on dieting. In reality, however, many people hesitate to disclose their own actual body shape or the like.

A purpose of the present disclosure is therefore to provide a group registration device, a group registration method, and a group registration program that can ease a user's hesitation in joining a group aiming for health promotion.

### Means for solving the problems

A group registration device of an aspect of the disclosure comprises: determination means for, based on physical information indicating a physical state of a user, determining a user type representing the physical state of the user from one or more predetermined points of view; and registration means for registering a user whose user type has been determined by the determination means for a group to which another user having a commonality belongs.

This configuration allows a user to indicate the user's own body shape or the like using a user type that represents it from one or more predetermined points of view instead of using concrete information such as weight, height, and body composition. The user then, by registering for a group to which another user having a commonality belongs, can do an action such as training to reach the user's target physical state while communicating with the other user. Note that the physical state is a user's metabolic age and health age, in addition to the user's height, weight, and body composition. The one or more points of view are a user's one or more pieces of physical information and an index such as metabolic age calculated based on the physical information as described in detail later, and a user type is determined by a piece of physical information or an index, or a combination of two or more pieces of physical information and indices. The commonality in a group is, for example, a similarity in physical information, a similarity in a goal, and a similarity in metabolic age.

As described above, a user is registered for a group under a determined abstract user type, and therefore can communicate with another user without the user's physical information being known directly to the other user. At the same time, a user type is determined based on a user's physical information, and is therefore credible to a certain extent. This configuration thus allows for easing a user's hesitation in joining a group aiming for health promotion.

In the above-described group registration device, the determination means, based on at least one or more pieces of the physical information chosen from a plurality of kinds of the physical information by a user, may determine as the user type one of subdivisions into which divisions each indicating a human body characteristic are subdivided for each division. This configuration allows a user to register for a group under a user type that the user imagines as the user's own.

In the above-described group registration device, the registration means may register a user for the group based on metabolic age calculated based on the user's physical information. This configuration allows, for example, users of the same metabolic age to form a group. Since metabolic age is an index that can be changed by a user making an effort, a user can be motivated to belong to a group for a young metabolic age.

In the above-described group registration device, the registration means may register a user for the group based on the user type determined by the determination means. This configuration allows users of the same user type to form a group, and facilitates communication with other users.

In the above-described group registration device, the group registration device may have goal setting means for setting a goal for the physical state of a user, and the registration means may register a user for the group based on the goal set by the goal setting means. This configuration allows users having the same goal to form a group, and facilitates communication with other users.

In the above-described group registration device, the determination means may determine the user type based on the physical information of a user sent from a measurement device. This configuration eliminates the need for a user to input the user's own physical information. In addition, since physical information is not input by a user, improper and fraudulent inputs can be prevented.

The above-described group registration device may have: goal setting means for setting a goal for the physical state of a user; and goal-achieving condition deriving means for deriving a condition for achieving the goal set by the goal setting means. This configuration allows a user to recognize what is required to achieve the user's own goal.

In the above-described group registration device, whether to allow the other user to read the user type determined by the determination means or not may be settable. This configuration prevents another user from getting to know even a user type representing the physical state of a user.

The above-described group registration device may have advice output means for outputting advice to improve the physical state of a user for each user type determined by the determination means. This configuration allows for outputting appropriate advice for each group and encouraging communication among users.

In the above-described group registration device, the advice output means may output advice to improve the physical state of a user for each user. This configuration allows for outputting advice appropriate for each user.

The above-described group registration device may have: goal setting means for setting a goal for the physical state of a user; and action deriving means for, based on the present physical information of a user and the set goal, deriving an action to be done by the user to achieve the goal. This configuration makes it easier for a user to achieve the user's own goal.

In the above-described group registration device, the action deriving means may derive a type and action frequency of the action based on the user's wish. This configuration allows for deriving an action more appropriate for a user.

In the above-described group registration device, the action deriving means may derive a type and action frequency of the action based on the user's lifestyle. This configuration allows for deriving an action more appropriate for a user.

In the above-described group registration device, the action deriving means may present the user with a predicted value of a change in the physical state of the user caused by the user doing the action. This configuration allows a user to recognize how the action will change the user's own physical state in the future.

The above-described group registration device may have: goal setting means for setting a goal for the physical state of a user; and rewarding means for giving a predetermined reward to a user when the user has achieved the goal. This configuration allows a user to be motivated to achieve a goal.

In the above-described group registration device, the determination means may determine the user type based on the present physical information of a user and the physical state aimed for by the user. This configuration prevents another user from getting to know a user's own present physical state even if the user permits the other user to read the user's own user type.

In the above-described group registration device, values of electrical resistance of a user's one or more body parts may be used to determine the user's sex. This configuration allows for the determination of a user type without a user entering the user's own sex.

A group registration method of an aspect of the disclosure has: a first step of, based on physical information indicating a physical state of a user, determining a user type representing the physical state of the user from one or more predetermined points of view; and a second step of registering a user whose user type has been determined by the first step for a group to which another user having a commonality belongs.

A group registration program of an aspect of the disclosure causes a computer to function as: determination means for, based on physical information indicating a physical state of a user, determining a user type representing the physical state of the user from one or more predetermined points of view; and registration means for registering a user whose user type has been determined by the determination means for a group to which another user having a commonality belongs.

### Advantage of the invention

The disclosure allows for easing a user's hesitation in joining a group aiming for health promotion.

### BRIEF DESCRIPTION OF THE DRAWINGS

Figure 1 shows a group system of an embodiment of the disclosure;
Figure 2 is a function block diagram showing an electrical configuration of a server of the embodiment of the disclosure;
Figure 3 is a flowchart showing a flow of a group registration process of the embodiment of the disclosure;
Figure 4 is a schematic diagram showing user types of the embodiment of the disclosure;
Figure 5 is a schematic diagram showing pieces of advice for each user type of the embodiment of the disclosure;
Figure 6 shows user registration images of the embodiment of the disclosure, where (A) is a user registration image that reflects user information and (B) is a user registration image that reflects a user type;
Figure 7 shows a task view image of the embodiment of the disclosure; and
Figure 8 shows an action balance setting image of the embodiment of the disclosure.

### MODES OF EMBODYING THE INVENTION

Embodiments of the disclosure will now be described with reference to the drawings. The embodiments described below are merely illustrative of ways to implement the disclosure, and do not limit the disclosure to the specific configurations described below. When the disclosure is to be implemented, any specific configuration may be appropriately adopted according to each embodiment.

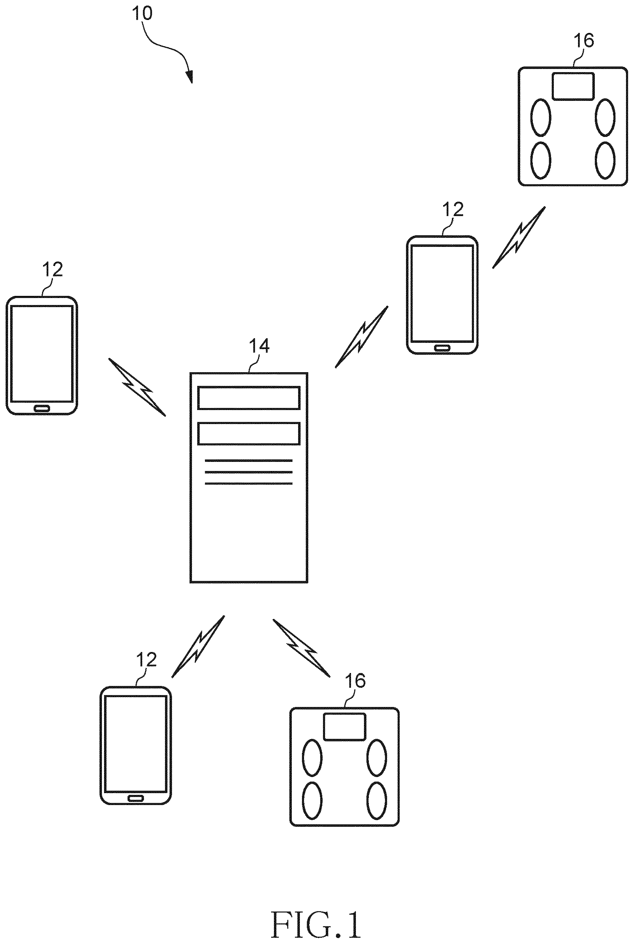

Figure 1 shows a group system 10 of an embodiment. The group system 10 of the embodiment comprises a plurality of portable terminal devices 12, a server 14, which is a group registration device, and a body composition analyzer 16, which is a measurement device for measuring physical information of a user.

Each portable terminal device 12 is an information processing device including, for example, a smartphone, a tablet terminal device, and a laptop personal computer. Each portable terminal device 12 is owned by each user who is in a group, and sends a variety of information on each user to the server 14. Each portable terminal device 12 is installed with application software (hereinafter referred to as the "group app") that allows for registration for a group in the embodiment, communication with another user in the group, reading and sending of information in the group, and the like. The information processing device to be installed with the group app may be a desktop personal computer instead of a portable terminal device 12.

The server 14 receives a variety of information on a user (hereinafter referred to as "user information") from each portable terminal device 12 and the body composition analyzer 16. User information includes a user's age, sex, physical information, and the like.

The body composition analyzer 16 is owned, for example, by a user, but may also be owned by a fitness club or the like. Measurements taken by the body composition analyzer 16 of the embodiment may be sent either to the server 14 via a portable terminal device 12 or to the server 14 directly from the body composition analyzer 16. When they are sent to the server 14 directly from the body composition analyzer 16, the same user ID as registered for a group is registered for the body composition analyzer 16.

The body composition analyzer 16 thus functions as a cooperation device for sending measured values of a user to the server 14 (hereinafter referred to as an "app cooperation device"). Measured values of a user may be inputted to a portable terminal device 12 by the user, and the input values may be sent to the server 14.

The server 14 of the embodiment registers a user for a group based on user information. The group is, for example, for improving metabolic age and carrying out appropriate health promotion such as dieting. A user registers for one of a plurality of kinds of groups that corresponds to the user's goal or the like, and carries out dieting or other health promotion while communicating with another user within the same group. In other words, a user acts to achieve a goal while cooperating with another user within the same group who has a shared sense and a shared style.

Now, when a user registers for a group, the user's own actual body shape and age may be required to be disclosed. In reality, however, many people hesitate to disclose their own actual body shape or the like.

The server 14 of the embodiment then, based on physical information indicating the physical state of a user, determines a user type (a super-deformed character etc.) representing the physical state of the user from one or more predetermined points of view, and registers a user whose user type has been determined for a group to which another user having a commonality belongs. Note that the physical state includes height, weight, and body composition. Weight and body composition are, for example, measurements taken by the body composition analyzer 16. Body composition is, for example, information on at least one of fat percentage, fat mass, fat-free mass, muscle mass, visceral fat mass, visceral fat level, visceral fat area, subcutaneous fat mass, basal metabolic expenditure, bone mass, body water percentage, BMI, intracellular fluid volume, and extracellular fluid volume. The one or more points of view are a user's one or more pieces of physical information and an index such as metabolic age calculated based on the physical information as described in detail later, and a user type is determined by a piece of physical information or an index, or a combination of two or more pieces of physical information and indices. The commonality in a group is, for example, a similarity in physical information, a similarity in a goal, and a similarity in metabolic age.

This configuration allows a user to indicate the user's own body shape or the like by a user type representing it abstractly, or euphemistically in other words, instead of concrete information such as weight, height, and body composition. Consequently, a user can communicate a feature and an image of the user's own body to another user with some objectivity without giving concrete numerical information. By registering for a group to which another user having a commonality belongs, a user can communicate with the other user and at the same time do, for example, training or the like to reach the physical state that the user aims at.

As described above, a user is registered for a group under a determined abstract user type, and therefore can communicate with another user without the user's physical information being known directly to the other user. At the same time, a user type is determined based on a user's physical information, and is therefore credible to a certain extent. The group system 10 of the embodiment thus allows for easing a user's hesitation in joining a group aiming for health promotion.

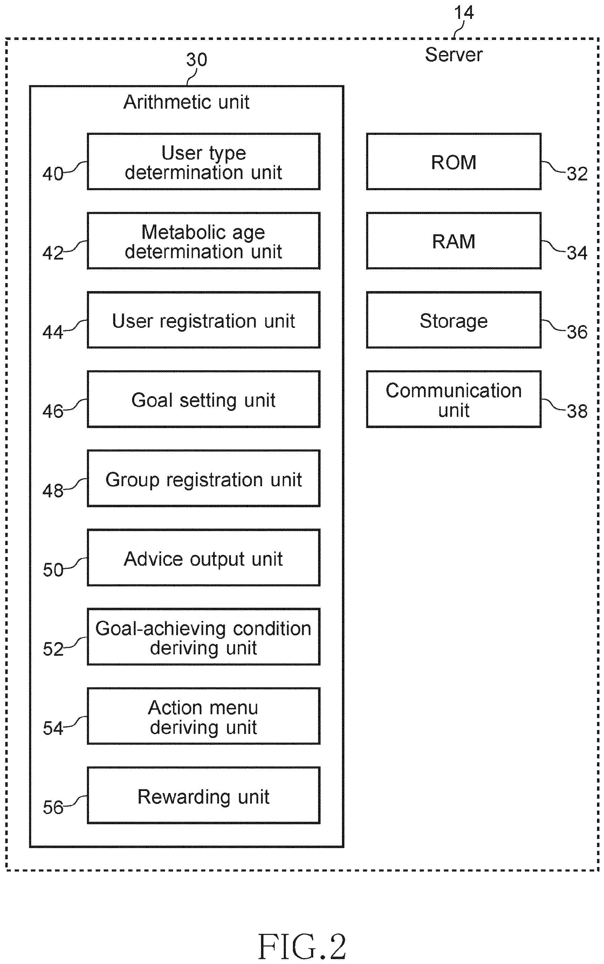

Figure 2 is a function block diagram showing an electrical configuration of the server 14 of the embodiment.

The server 14 of the embodiment comprises: an arithmetic unit 30 comprising a CPU (Central Processing Unit) or the like; a ROM (Read Only Memory) 32 stored in advance with various programs, various items of data, and the like; a RAM (Random Access Memory) 34 to be used as a work area or the like when the arithmetic unit 30 executes various programs; a storage 36 stored with various programs and various items of data; and a communication unit 38 for sending and receiving data to and from the portable terminal devices 12, the body composition analyzer 16, and the like.

The arithmetic unit 30 of the embodiment comprises a user type determination unit 40, a metabolic age determination unit 42, a user registration unit 44, a goal setting unit 46, a group registration unit 48, an advice output unit 50, a goal-achieving condition deriving unit 52, an action menu deriving unit 54, and a rewarding unit 56. In the embodiment, each function that the arithmetic unit 30 has is implemented by, for example, the arithmetic unit 30 executing a program stored in the storage 36. The embodiment is not limited to this, and each function may be implemented by separate hardware such as an ASIC (Application Specific Integrated Circuit) of the server 14.

The user type determination unit 40, based on physical information indicating the physical state of a user, determines a user type abstractly representing the user's physical state.

The metabolic age determination unit 42 determines the metabolic age of a user based on the user's physical information. The metabolic age can be obtained by determining which age, body composition, or basal metabolism a user's result of measurement of muscle development, basal metabolism, or the like is close to.

The user registration unit 44 registers a user whose user type has been determined.

The goal setting unit 46 sets a goal for the physical state of a user (hereinafter referred to as a "user goal"). A user goal may be not just a user's body shape or weight, but also metabolic age or health age, or a group that a user wants to join.

The group registration unit 48 registers a user whose user type has been determined by the user type determination unit 40 for a group to which another user having a commonality belongs.

The advice output unit 50 outputs advice to improve the physical state of a user for each user type determined by the user type determination unit 40. The advice output unit 50 may output advice to improve the physical state of a user for each user.

The goal-achieving condition deriving unit 52 derives a condition for achieving a user goal set by the goal setting unit 46.

The action menu deriving unit 54, based on present physical information of a user and a set user goal, derives an action to be done by the user to achieve the user goal (hereinafter referred to as an "action menu"). An action menu includes, for example, the details of an exercise or a meal.

The rewarding unit 56 gives a predetermined reward to a user when the user has achieved a user goal.

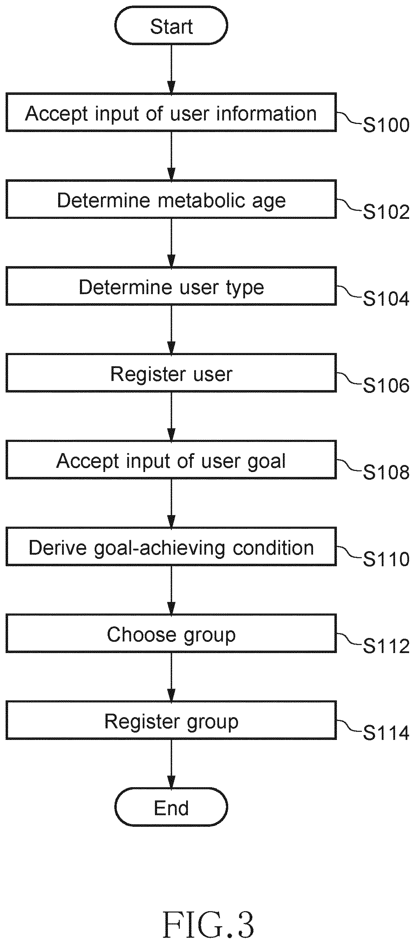

Figure 3 is a flowchart showing a flow of a group registration process (a group registration program) to be executed by the server 14. The group registration process is started by a user launching the group app installed on the user's own portable terminal device 12 and sending user information to the server 14.

First at a step S100, an input of user information is accepted from the portable terminal device 12 via the communication unit 38. Note that user information includes an e-mail address, an address, a nickname, a hometown, a photograph of a hobby, and a photograph of the user's face, in addition to the user's physical information described above. Whether to input these pieces of information or not may be determined arbitrarily by the user.

The user also inputs whether or not the user owns the body composition analyzer 16, which is an app cooperation device. If the user owns the body composition analyzer 16, which is an app cooperation device, the user sends specific information on the body composition analyzer 16 to the server 14 to enable the cooperation. Consequently, each time the user uses the body composition analyzer 16 to measure the user's own weight and body composition, the measurement result is sent to the server 14 and the user's physical information is successively updated. As described later, the user type determination unit 40 then determines the user's user type based on the user's physical information sent from the body composition analyzer 16. This eliminates the need for the user to input the user's own physical information. In addition, since physical information is not input by the user, improper and fraudulent inputs can be prevented.

At the next step 102, the metabolic age determination unit 42 determines the user's metabolic age based on the user's physical information.

At the next step 104, the user type determination unit 40 determines the user type based on the user's physical information.

The user type determination unit 40 of the embodiment, based on one or more pieces of physical information chosen from a plurality of kinds of physical information by the user, determines as the user type one of subdivisions into which divisions each indicating a human body characteristic (hereinafter referred to as "body shape divisions") are subdivided for each division.

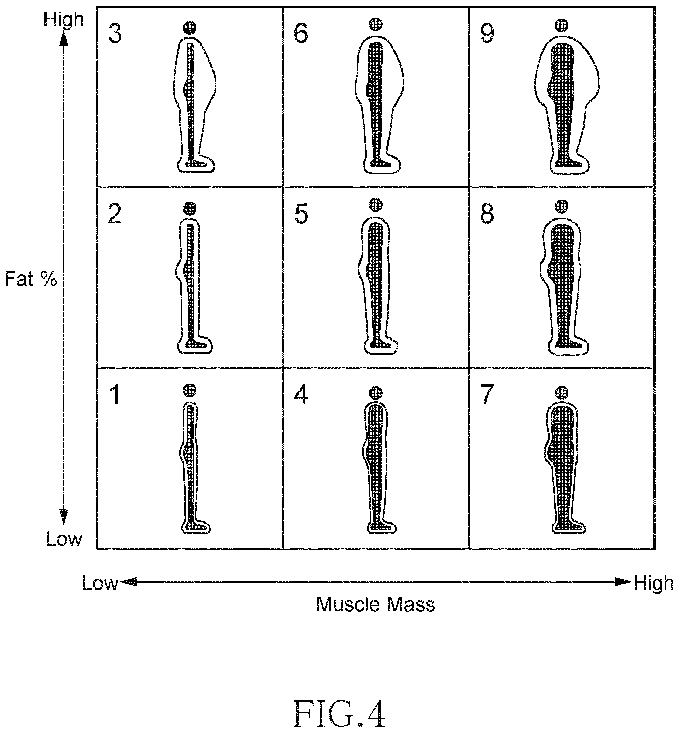

Now, Figure 4 is a schematic diagram showing an example of user types of the embodiment. Figure 4 provides body shape divisions that are a combination of fat percentage and muscle mass, where the body shape divisions are subdivided into a matrix of nine subdivisions according to the levels of fat percentage and muscle mass.

Referring to the example in Figure 4, the user type determination unit 40 extracts the user's fat percentage and muscle mass from the user's physical information, and determines as the user's user type one of the nine determination boxes subdivided in the form of a matrix. The example in Figure 4 provides the types as follows: "1" for thin; "2" for underexercised; "3" for hidden obese; "4" for thin and muscular; "5" for standard; "6" for obese; "7" for very muscular; "8" for muscular; and "9" for solidly-built. Note that the subdivided determination boxes shown in Figure 4 are just an example, and the subdivision may be finer than nine.

Body shape divisions may be not just the combination of fat percentage and muscle mass, but also a combination of basal metabolism and visceral fat, a combination of fat percentage, muscle mass, basal metabolism, and visceral fat, or another combination of bone mass, basal metabolism, body water, muscle quality score, leg muscle score, metabolic age, health age, and the like. The combination is, for example, chosen by the user. The user, for example, imagines a group that the user wants to join and chooses physical information for determination of the user type. Such user type determination allows the user to register for a group under a user type that the user imagines as the user's own.

Upon the determination of the user type, the advice output unit 50 outputs the determined user type and advice to improve the physical state of the user for each user type, and presents them to the user. To present here means to display on a screen of the user's portable terminal device 12.

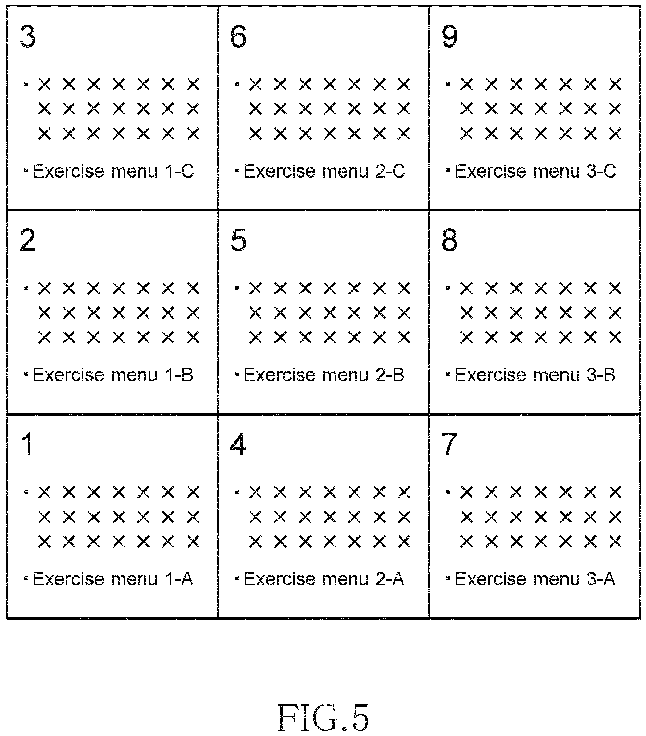

An example of the advice will be described with reference to Figure 5. A matrix shown in Figure 5 corresponds to the matrix shown in Figure 4. A description of each user type and an exercise menu as advice are presented in each one of boxes "1" through "9" of the matrix shown in Figure 5. The advice is, for example, the same for each user type, and is therefore shared with another user within a group of the same user type. The advice may be not just an exercise menu, but also the details of a meal, life habits, and the like.

The embodiment is not limited to this, and advice to improve the physical state of the user may be output for each user. Advice is output in this mode, for example, based not just on the physical information used to determine the user type but also on physical information not used for the determination or on a user goal. As a result, advice is output appropriately for each user.

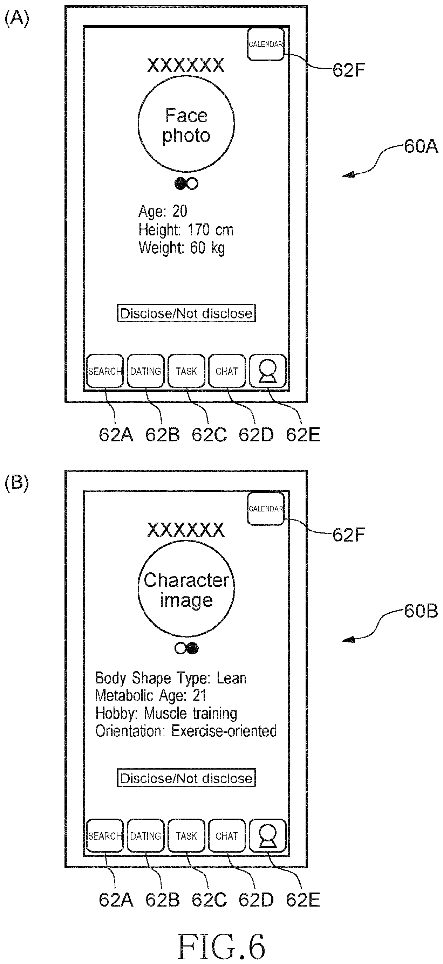

At the next step 106, the user registration unit 44 registers the user, whose user type has been determined, as a user capable of joining a group. Figure 6 shows user registration images 60A and 60B of the embodiment. Figure 6(A) shows the user registration image 60A, which directly reflects the input user information. Figure 6(B) shows the user registration image 60B, which reflects the user type.

A nickname for distinguishing the user is displayed in "XXXXXX" in the user registration images 60A and 60B.

A character image that is, for example, suggestive of the user type is displayed in "Character Image" in the user registration image 60B. The character image may be, for example, a human-like image as shown in Figure 4, or a plurality of images may be set for each user type and the user may choose one of them. The character image may also be, for example, an image that represents an anime character or entertainer corresponding to the user type.

"Body Shape Type" in the user registration image 60B is a designation suggestive of the user type, and "Lean" shown in Figure 6(B) is the designation of the very muscular type, which is "7" in Figure 4. The designation is predetermined for each user type. "Metabolic Age" in the user registration image 60B is a result of determination made by the metabolic age determination unit 42. Moreover, "Hobby" and "Orientation" in the user registration image 60B are pieces of information input as the user information.

Whether to allow another user to read or not (Disclose/Not disclose) is set for both user registration images 60A and 60B. That is, some users may want another user not to know even a determined user type. For this reason, whether to allow another user to read a user's own user type or not is settable in the embodiment. Consequently, a user who has set not to allow reading (Not disclose) can prevent another user from getting to know even a user type, which is the user's own abstract physical information.

The embodiment is not limited to this, and another user's information that a user is allowed to read with the user's right may change. For example, another user is allowed to set which information can be read by a user with charge, points, or the like. This allows a user to be given the right to read another user's information with charge, points, or the like.

User registration is done by the user agreeing to the user registration images 60A and 60B shown in Figure 6. If, for example, the user does not agree to the user's own user type or the like, the process returns to the step 104, physical information other than the last one is chosen, and a user type is determined again.

At the next step 108, an input of a user goal is accepted from the portable terminal device 12 via the communication unit 38, and the goal setting unit 46 sets the user goal. The user goal may also be set to be a group that the user wants to join or a character or an entertainer whom the user wants to become, in addition to the above-described body shape, metabolic age, or the like aimed for by the user.

At the next step 110, the goal-achieving condition deriving unit 52 derives a goal-achieving condition for achieving the user goal. An example of calculating a goal-achieving condition will be described below.

The goal-achieving condition deriving unit 52 calculates, for example, the shortest distance (e.g., the Euclidean distance) from the coordinates of the user's present body composition to the coordinates that indicate a target body shape that has been input as the user goal. Then, based on the calculation result, the goal-achieving condition deriving unit 52 calculates a specific target amount of change required for the user to reach the target body shape and presents it to the user by showing something like, "Lose 1 kg of fat and gain 0.8 kg of muscle." Similarly, in regard to a target metabolic age, the goal-achieving condition deriving unit 52 calculates a specific target amount of change based on the difference between the user's present metabolic age and the target metabolic age and presents it to the user by showing something like, "Lose 1.5 kg of fat and gain 0.8 kg of muscle."

In addition, the goal-achieving condition deriving unit 52 may accept an input of a period for achieving the goal or a date. The goal-achieving condition deriving unit 52 then calculates a daily target amount of change based on the target period or days to the target date and presents it to the user by showing something like, "Lose -50 g of fat per day and gain 10 g of muscle per day."

Since a goal-achieving condition for achieving a user goal is thus presented to a user in the embodiment, the user can recognize what is required to achieve the user's own goal. The user may therefore revise and reenter the user goal after seeing the presented goal-achieving condition. Note that the user can see the goal-achieving condition even after being registered for a group.

At the next step 112, the user registration unit 44 presents the user with groups estimated to be appropriate for the user, and the user chooses one or more groups from the presented ones.

Note that a group may be registered based on, for example, the determined user type, the user goal, or the determined metabolic age. In addition, the user can choose a group also with reference to whether an index behind the grouping relates to appearance or not. As just described, the embodiment allows for forming a group of users of the same user type, users having the same goal, or users of the same metabolic age, and therefore facilitates communication among the users. Within a group based on metabolic age in particular, even users of different actual ages can communicate with other users in an age bracket which they entered with their own efforts regardless of their actual ages.

Groups are not limited to the above, and may be based on, for example, the user's lifestyle. A group based on the lifestyle is a group into which a user is categorized by measurable things of daily life including, for example, the user's sleeping hours, wake-up time and bedtime, the speed of eating a meal, and favorite seasoning. If a user is to be registered for a group like this, the user is required to input these pieces of information in advance as user information. The lifestyle of the user may be acquired from a wearable device worn by the user. Such lifestyle-based grouping facilitates communication within the group.

In addition, a group may be identified by a designation, and the designation of a group may be suggestive of an outline of the group and may give a sense of affinity or fun to users.

If the user thinks that a group appropriate for the user is not suggested, the process then, for example, may return to the step 108, where the user may input a user goal different from the last time and may thereby cause a group different from the last time to be suggested. Moreover, the user may create a new group corresponding to the user type or user goal on the user's own. A group newly created by the user is registered on the server 14.

At the next step 114, the group registration unit 48 registers the group chosen by the user as the group that the user is in. This causes the user to act to achieve the user's own goal while communicating with another user within the registered group.

Communication with another user in the registered group is done by operating various buttons 62A to 62D shown in Figure 6.

The button 62A is operated to look for another user registered for the same group as the user, and another user with whom the user wants to communicate is searched for by entering a keyword or the like.

The button 62B is for getting fixed up with a person of the opposite sex. The user can get fixed up with a person of the opposite sex with the user's ideal body shape as an example of a reward for the user achieving the user's own user goal. The user therefore inputs information on a person of the opposite sex with the user's ideal body shape in advance as the user's own user information. A person of the opposite sex to be fixed up is another user registered via the group app, and selection of a user to be fixed up is done by using body shape information based on body composition or other physical information, and is therefore credible to a certain extent. The reward is not limited to the above, and may also be, for example, a predetermined commodity, points that can be exchanged for a certain commodity or service, or the like.

The information on the user's ideal person of the opposite sex may include a personality. For example, a personality may be determined from data from a body composition analyzer, the state of implementation or continuation of a task, how to use the app, or the like, and a result of such determination may be added to a profile of a person of the opposite sex with an ideal body shape.

The button 62C is operated to implement a task. The task is, for example, the user's implementation of an exercise menu or the like for achieving the user goal.

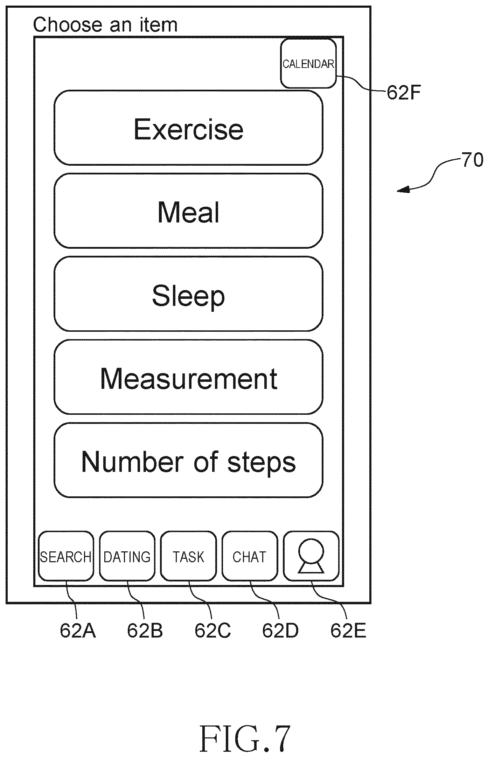

Now, for example, the user's operating the button 62C causes the display of a task view image 70 shown in Figure 7. The user's further operating a button of each item causes the display of a task of the operated item.

For example, operating an item "Exercise" in Figure 7 causes the display of a menu of exercises to be done in the day. This exercise menu may be, for example, set for each user, or displayed for each user type as advice to improve the physical state of the user (see Figure 5). Operating an item "Meal" causes the display of the details of and/or recipes for meals to be consumed in the day. Operating an item "Sleep" causes the display of bedtime and wake-up time of the day. Operating an item "Measurement" causes the display of a date and time for the next measurement of the user's physical information and/or the entry screen for body composition or the number of steps. Operating an item "Number of steps" causes the display of the number of steps to be achieved in a day. How to set each item will be described later as the balance setting. In addition, operating the button 62C may cause the display of a list of tasks other than the example in Figure 7.

The button 62C may be operated not only when tasks are to be viewed but also when the user wants to implement an identical exercise menu together with another user who belongs to the same group. For example, the user operates the button 62C to call another user, and does an exercise displayed by the item "Exercise" together with the other user. The button 62D is operated to chat with another user in the same group. In this way, the group app of the embodiment allows the user to carry out dieting or other health promotion together with another user who belongs to the same group or, in other words, another user having a common awareness such as the same goal while communicating with the other user through exercise or the like.

The group app of the embodiment may be provided with a function to set the user's schedule to do exercise together with another user. In this mode, the group app is launched at a scheduled date and time, and facilitates a plurality of users' doing exercise together.

The button 62E is for viewing a variety of personal information of the user, and the user's operating the button 62E causes, for example, the presentation of the goal-achieving condition and a later-described action menu of the user's own.

In the embodiment, the user type may be determined each time the user's physical information is newly input. That is, the user type changes successively with the actual physical state of the user. In that case, the group for which the user is registered may change with the change of the user type, or may remain unchanged while the user type changes.

An action to be done by a user using the group app of the embodiment (an action menu) will be described next. An action menu includes the details of an exercise, a meal, and the like.

As described before, an action that a user does to achieve a user goal is derived by the action menu deriving unit 54 based on the user's present physical information and set user goal.

The action menu deriving unit 54 of the embodiment derives the type and action frequency of an action menu based on the user's wish. The term action frequency is, for example, the number and duration of exercises in a day as for exercises, and the quantity and number of meals as for meals. That is, the action menu deriving unit 54 derives an action menu based on not just the physical information and user goal input by the user, but also the balance setting which indicates the allocation between exercises, meals, and the like in accordance with the user's wish. The action menu deriving unit 54 can thus derive an action menu more appropriate for the user.

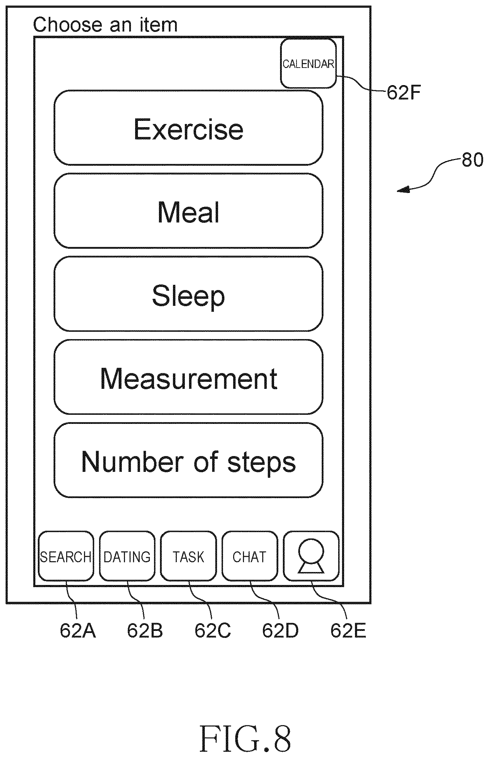

Figure 8 shows an action balance setting image 80 of the embodiment. Items in the action balance setting image 80 are, for example, the same as those in the task view image 70 shown in Figure 7. The user determines the allocation in an action menu via the action balance setting image 80. For example, the user clicks "Exercise" in Figure 7 and inputs a numerical value, thereby increasing or decreasing the percentage of exercises as an action menu for achieving the user goal, or clicks "Meal" and inputs a numerical value, thereby increasing or decreasing the percentage of meals as an action menu. This, for example, allows the user to determine an action menu so as to achieve the user goal by increasing the percentage of dietary restriction more than that of exercises, or by increasing the percentage of exercises more than that of dietary restriction.

The action menu deriving unit 54 of the embodiment may derive an action menu also with reference to the user's lifestyle. For example, the user may input days of the week on which the user works, and the action menu deriving unit 54 may be set to derive an identical action menu regardless of whether it is a weekday with work or a holiday without work. The embodiment is not limited to this, and the action menu deriving unit 54 may be set to derive an action menu that offers more exercises on holidays than on weekdays or that offers more exercises on weekdays than on holidays. In addition, the action menu deriving unit 54 may be set to derive an action menu that offers more exercises on long vacations.

The action menu deriving unit 54 may also be set to offer a bedtime exercise based on an input of the user's sleep window. Based on an input of the number of steps in a day, the action menu deriving unit 54 may also be set to include this number of steps in an action menu. Based on an input of the timing of measuring the user's body composition, the action menu deriving unit 54 may also be set to derive an action menu that accords with the measurement timing. These setting items may be sent from another device (a wearable device or the like) in advance instead of being input by the user on the user's own.

Moreover, the action menu deriving unit 54 presents the user with the predicted value of a change in the physical state of the user caused by the user doing an action menu. The action menu deriving unit 54, in the process of deriving an action menu, is to also derive a change in the physical state of the user caused by the user doing the action menu. The action menu deriving unit 54 of the embodiment therefore presents the user with the predicted value of a change in the physical state of the user based on an action menu by causing the user's portable terminal device 12 to display it as an ideal graph. This allows the user to recognize a change in the user's own physical state caused by doing the action menu in the future.

More specifically, a daily action menu and an ideal graph are displayed on the portable terminal device 12 by the user operating a calendar button 62F on the images of the group app displayed on the portable terminal device 12. An actually measured value of the user's physical state of the day may be displayed on (written over) the calendar.

When the user has at last achieved the user's own user goal, the rewarding unit 56 gives a predetermined reward to the user as described above. This allows the user to be motivated to achieve the goal.

While the disclosure has been described with reference to the above embodiments, the technical scope of the disclosure is not limited to the scope provided by the embodiments. Various modifications or improvements can be made to the embodiments without departing from the gist of the disclosure, and those added with the modifications or improvements are also included in the technical scope of the disclosure.

For example, while a description has been made for the above embodiments on a mode in which a user type is determined based on the present physical information of a user, the disclosure is not limited to this. For example, a user type may be determined based on the present physical information of a user and the physical state aimed for by the user (a user goal). That is, a user type indicates the physical state aimed for by a user instead of the present physical state of the user. This prevents another user from getting to know a user's own present physical state even if the user permits the other user to read the user's own user type.

While a description has been made for the above embodiments on a mode in which a user enters the user's own sex as user information, the disclosure is not limited to this. For example, the server 14 may use the values of electrical resistance of a user's one or more body parts to determine the user's sex. This allows for the determination of a user type without a user entering the user's own sex. For this purpose, for example, the storage 36 holds a regression equation for each sex. These regression equations are created by carrying out measurement of various people's bodies including, for example, measurement of values of electrical resistance between predetermined parts of their bodies, in order to obtain body composition.

While a description has been made for the above embodiments on a mode in which a user's metabolic age is calculated and used for a user goal and group registration, the disclosure is not limited to this. In addition to a user's metabolic age, not a user's actual age but another index indicating an age calculated from a user's physical state, such as a user's health age, may be calculated and used for a user goal and group registration.

While a description has been made for the above embodiments on a mode in which the body composition analyzer 16, as a cooperation device, sends measured values of a user to the server 14, the disclosure is not limited to this. A blood pressure monitor, a salinometer, various types of timers, and other devices for measuring the lifestyle of a user may be applied as cooperation devices, and these cooperation devices may send measured values to the server 14 for use in the determination of a user type, group registration, and the like performed by the server 14.

The user type determination unit 40 may differently treat physical information that has been manually input by a user and physical information that has been input by acquiring from the body composition analyzer 16 or other measurement devices. For example, the user type determination unit 40 may determine a user type in such a way that different user types are determined from physical information that has been manually input by a user and physical information that has been input by acquiring from a measurement device.

The group registration unit 48 may register a user for a group on the assumption that different user types are determined from physical information that has been manually input by a user and physical information that has been input by acquiring from the body composition analyzer 16 or other measurement devices. In addition, a group may be set up that only users whose physical information has been input from the body composition analyzer 16 or other measurement devices can belong to, and only users whose physical information has been input from a measurement device may be allowed to communicate with users whose physical information has been input from a measurement device. Manual input can be made fraudulently, whereas physical information that has been input from a measurement device is information actually received therefrom, and therefore this configuration allows for communication with users whose physical information is highly credible. Moreover, whether a service with this configuration is to be provided or not may be determined according to whether the user has agreed to be charged or not. Since charged users are allowed to select and communicate with users with highly credible physical information, different services can be developed for uncharged and charged users.

### DESCRIPTION OF THE SYMBOLS

10: Group system
12: Portable terminal device
14: Server (Group registration device)
16: Body composition analyzer (Measurement device)
40: User type determination unit (Determination means)
46: Goal setting unit (Goal setting means)
48: Group registration unit (Registration means)
50: Advice output unit (Advice output means)
52: Goal-achieving condition deriving unit (Goal-achieving condition deriving means)
54: Action menu deriving unit (Action deriving means)
56: Rewarding unit (Rewarding means)

## Claims

1. A group registration device comprising:
determination means for, based on physical information indicating a physical state of a user, determining a user type representing the physical state of the user from one or more predetermined points of view; and
registration means for registering a user whose user type has been determined by the determination means for a group to which another user having a commonality belongs.

2. The group registration device according to claim 1, wherein the determination means, based on one or more pieces of the physical information chosen from a plurality of kinds of the physical information by a user, determines as the user type at least one of subdivisions into which divisions each indicating a human body characteristic are subdivided for each division.

3. The group registration device according to claim 1 or 2, wherein the registration means registers a user for the group based on metabolic age calculated based on the user's physical information.

4. The group registration device according to any one of claims 1 to 3, wherein the registration means registers a user for the group based on the user type determined by the determination means.

5. The group registration device according to any one of claims 1 to 4, having goal setting means for setting a goal for the physical state of a user, wherein the registration means registers a user for the group based on the goal set by the goal setting means.

6. The group registration device according to any one of claims 1 to 5, wherein the determination means determines the user type based on the physical information of a user sent from a measurement device.

7. The group registration device according to any one of claims 1 to 6, having:
goal setting means for setting a goal for the physical state of a user; and
goal-achieving condition deriving means for deriving a condition for achieving the goal set by the goal setting means.

8. The group registration device according to any one of claims 1 to 7, wherein whether to allow the other user to read the user type determined by the determination means or not is settable.

9. The group registration device according to any one of claims 1 to 8, having advice output means for outputting advice to improve the physical state of a user for each user type determined by the determination means.

10. The group registration device according to claim 9, wherein the advice output means outputs advice to improve the physical state of a user for each user.

11. The group registration device according to any one of claims 1 to 10, having:
goal setting means for setting a goal for the physical state of a user; and
action deriving means for, based on the present physical information of a user and the set goal, deriving an action to be done by the user to achieve the goal.

12. The group registration device according to claim 11, wherein the action deriving means derives a type and action frequency of the action based on the user's wish.

13. The group registration device according to claim 11 or 12, wherein the action deriving means derives a type and action frequency of the action based on the user's lifestyle.

14. The group registration device according to any one of claims 11 to 13, wherein the action deriving means presents the user with a predicted value of a change in the physical state of the user caused by the user doing the action.

15. The group registration device according to any one of claims 1 to 14, having:
goal setting means for setting a goal for the physical state of a user; and
rewarding means for giving a predetermined reward to a user when the user has achieved the goal.

16. The group registration device according to any one of claims 1 to 15, wherein the determination means determines the user type based on the present physical information of a user and the physical state aimed for by the user.

17. The group registration device according to any one of claims 1 to 16, wherein values of electrical resistance of a user's one or more body parts are used to determine the user's sex.

18. A group registration method having:
a first step of, based on physical information indicating a physical state of a user, determining a user type representing the physical state of the user from one or more predetermined points of view; and
a second step of registering a user whose user type has been determined by the first step for a group to which another user having a commonality belongs.

19. A group registration program for causing a computer to function as:
determination means for, based on physical information indicating a physical state of a user, determining a user type representing the physical state of the user from one or more predetermined points of view; and
registration means for registering a user whose user type has been determined by the determination means for a group to which another user having a commonality belongs.
